# DEMANDE DE BREVET EUROPEEN

(11) **EP 0 842 647 A1**
(43) Date de publication de la demande: **20.05.1998**
(21) Numéro de dépôt: 97420210.3
(22) Date de dépôt: 12.11.1997
(51) Int. Cl.: A61F 13/02

(54) **Dispositif occlusif adhésif permettant de dispenser differents produits actifs après pose sur la peau**

(30) Priorité: 13.11.1996 FR 9614044
(71) Demandeur: Maciocia, Yves, 69330 Pusignan (FR)
(72) Inventeur: Maciocia, Yves, 69330 Pusignan (FR)

(57) **Abrégé**

Dispositif occlusif adhésif permettant de dispenser, après pose sur la peau, des produits actifs ; caractérisé en ce que le dispositif possède une zone adhérente à la peau (1), la partie supérieure du pansement (4) possède une orifice obturable (3) permettant le remplissage ou la vidange en produit actif du réceptacle du dispositif (2).

Applications: Domaine Médical, Paramédical et Esthétique.

## Description

La présente invention concerne un dispositif pour conserver un produit liquide ou solide en contact avec la peau pendant une durée de plusieurs jours, de façon à favoriser son absorption par la peau. Plus précisément, le dispositif conforme à l'invention est destiné à permettre le contact de substances actives (sous forme de crème, gel, pommade, voire même solide) avec la peau.

Traditionnellement, les pansements occlusifs sont réalisés avec une gaze, un pansement ou un revêtement plastifié pour favoriser l'occlusion ; disposés, après étalement plus ou moins important de gel, pommade ou crème, en recouvrement et solidarisé par des bandes collantes en périphérie.
D'autre part, on connaît à ce jour des pansements possédant des produits actifs déterminés, mélangés ou non, à la masse adhésive destinée à coller le pansement sur la peau. Ces produits sont communément appelés << patchs >> ou << pansements transdermiques >>.

Dans le cas des pansements occlusifs, le fait d'appliquer certains gels ou pommades sur la peau puis d'essayer de circonscrire et de recouvrir la zone à traiter par un film plastique présente les inconvénients suivants :
- la zone concernée par la pommade ou le gel appliqués à la main est irrégulière et permet peu de quantifier la zone cutanée concernée par le passage du gel pouvant ainsi gêner la qualité du traitement par un excès ou un défaut de surface cutanée concernée.
- L'adhésivité marginale pour faire adhérer le pansement occlusif est très aléatoire, en effet, toute zone concernée par le gel sera rendue non adhérente et permettra la fuite du produit actif ainsi qu'un contact de celui-ci avec l'air ambiant, ce qui est contraire à la volonté d'occlusion.
- Enfin, le contact du produit actif avec la masse adhésive du produit utilisé pour solidariser ce pansement sur la peau, peut très facilement provoquer des allergies.

En ce qui concerne les << patchs >>, ces problèmes sont évités, mais ils ne permettent d'utiliser que le produit actif proposé par le fabricant et pour l'instant, la quantité de ces produits finis est limitée par rapport au nombre de produits actifs proposés en tube, flacons pressurisés, ou non, proposés sur le marché.

La présente invention vise à pallier ces différents inconvénients. Elle concerne un dispositif occlusif adhésif permettant d'appliquer, après pose sur la peau, divers produits actifs.

Ce descriptif se caractérise en ce que, après avoir collé ledit dispositif sur l'endroit choisi, une partie de ce dispositif est destinée à recevoir le produit actif.
- Typiquement la partie adhérente du dispositif est conventionnelle et marginale par rapport à la partie réceptacle du produit actif ; la partie réceptacle reçoit sur sa partie supérieure un orifice obturable permettant le remplissage du dispositif après pose sur la peau, ou le retrait du produit actif à volonté.
- Dans une forme de réalisation particulière, la partie réceptacle du produit est munie d'un cathéter permettant le remplissage ou la vidange du réceptacle à l'aide d'une seringue.

La manière dont l'invention peut être réalisée, et les avantages qui en découlent, ressortiront mieux des exemples de réalisations qui suivent, donnés à titre indicatif mais non limitatif, à l'appui des figures annexées.

La figure 1 représente une vue schématique en coupe du dispositif, selon une forme de réalisation de l'invention.

Les figures 2 et 3 représentent une vue schématique en coupe d'autres formes de réalisation de l'invention.

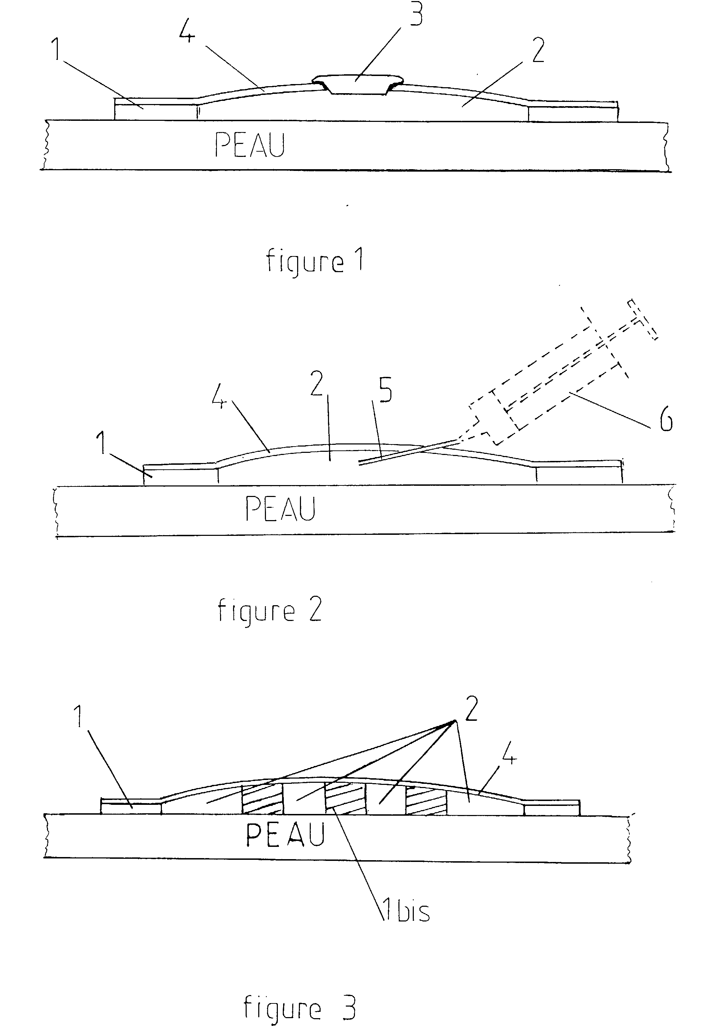

Comme on peut le voir dans la figure 1, dans laquelle on a représenté une vue schématique en coupe du dispositif de l'invention, celui-ci comporte une partie adhérente à la peau (1), un réceptacle (2) alimentable par une trappe (3) situé sur une partie supérieure (4) qui recouvre toute la surface du dispositif. Cette trappe (3) peut s'ouvrir ou se fermer à volonté pour alimenter ou désalimenter la partie réceptacle du dispositif (2), et ce, même une fois le dispositif mis en place sur la peau.

Dans une autre forme de réalisation, représentée par la figure 2, on retrouve la partie destinée à l'adhérence sur la peau (1) et le réceptacle (2), muni d'un cathéter (5). Ce cathéter (5) traverse la partie supérieure (4) qui recouvre toute la surface du dispositif et alimente ou vide le réceptacle (2). On a matérialisé la seringue (6) qui alimente ou désalimente le réceptacle du dispositif (2) par l'intermédiaire du cathéter.

Dans une 3ème forme de réalisation, non représentée, c'est toute la partie supérieure du dispositif qui est amovible, permettant ainsi de remplir ou de vider le réceptacle, une fois le dispositif collé sur la peau.

Dans une 4ème forme de réalisation, représentée par la figure 3, les zones adhérentes marginales (1) sont renforcées par des zones d'adhérence en matériau lacunaire et cotant sur ses 2 faces supérieure et inférieure (mousse autocollante, par exemple) (1 bis). L'aspect lacunaire de ce matériau délimite ainsi un réceptacle (2) qui permet de doser la quantité de produit actif et de délimiter la surface de peau traitée. Dans ce cas de figure, la partie supérieure du système (4) est amovible, de façon à pouvoir remplir le réceptacle (2) puis refermer pour rendre l'ensemble occlusif.

Dans une 5ème forme de réalisation, la partie supérieure amovible (5) est recouverte d'un matériau conducteur (film graphité par exemple) pour pouvoir ioniser le produit à l'intérieur du réceptacle.

Dans une 6ème forme de réalisation, les zones d'adhérence en matériau collant sur leurs 2 faces supérieure et inférieure (1 bis) peuvent être enduites d'un catalyseur ou d'un produit exothermique ou endothermique pour favoriser le passage du produit actif à travers la peau.

Dans une 7ème forme de réalisation, la partie supérieure amovible(4) est conçue dans un matériau réalisant une occlusion partielle.

De la sorte, on obtient des dispositifs:
- bien adhérents sur la peau,
- délimitant de façon précise la surface cutanée concernée,
- ne permettant pas le mélange produit actif/produit adhérent,
- remplissables ou vidangeables à volonté, et
- pouvant utiliser tout produit actif, liquide ou solide, selon les formes de réalisation.

De plus, du fait même de la possibilité de remplissage ou de vidange de la zone réceptacle, la pose du dispositif peut être prolongée sans l'effet d'abrasion cutanée due à la pose et au retrait des pansements occlusifs classiques ou des << patchs >>. Un tel dispositif est particulièrement adapté :
- pour réaliser des soins ambulatoires (anti-inflammatoire à demeure chez les sportifs, par exemple);
- pour éviter la pose et le retrait successifs de pansements qui altèrent l'état cutané déjà déficient de certains types de peau (peau des personnes âgées, par exemple) ;
- pour modifier le traitement local selon les résultats obtenus sans changer le dispositif.

La parfaite étanchéité obtenue évite la salissure des vêtements ou des draps.

La fabrication de tels dispositifs est conventionnelle et ne nécessite pas de mise en oeuvre spécifique.

## Revendications

1. Dispositif occlusif adhésif permettant de dispenser, après pose sur la peau des produits actifs ; caractérisé en ce que la partie supérieure du pansement possède un orifice obturable permettant le remplissage ou la vidange en produit actif de la partie réceptacle de ce dispositif.

2. Dispositif occlusif adhésif selon la revendication 1, caractérisé en ce que l'orifice supérieur du dispositif est constitué, par tout ou partie, d'un cathéter pouvant être en continuité fonctionnelle avec une seringue.

3. Dispositif occlusif adhésif, caractérisé en ce que le dispositif est constitué d'une partie collée sur la peau et délimitant la zone à traiter et d'une partie supérieure amovible réalisée en matériau occlusif. Le produit actif est appliqué sur la zone cutanée délimitée par la partie posée sur la peau puis la partie supérieure est appliquée pour obturer l'ensemble. Cette partie peut être partiellement solidaire ou non du reste du dispositif.

4. Dispositif occlusif adhésif selon la revendication 3, caractérisé en ce que la partie supérieure amovible vient après remplissage du réceptacle se coller sur un matériau lacunaire et adhérent sur ses 2 faces : 1 face/peau, 1 face/partie supérieure du dispositif.

5. Dispositif occlusif adhésif selon la revendication 3 et 4, caractérisé en ce que la partie supérieure est conductrice du courant électrique pour permettre une ionisation du produit actif contenu dans le réceptacle.

6. Dispositif occlusif adhésif selon la revendication 4, caractérisé en ce que le matériau lacunaire est imprégné d'un produit catalyseur ou exothermique ou encore endothermique.

7. Dispositif occlusif adhésif selon les revendications 1, 2, 3, 4, 5 et 6, caractérisé en ce que la partie supérieure du dispositif est conçue dans un matériau réalisant une occlusion partielle.
